# EUROPEAN PATENT APPLICATION

(11) **EP 4 792 871 A1**
(43) Date of publication of application: **19.08.2026**
(21) Application number: 24876370.8
(22) Date of filing: 23.09.2024
(51) Int. Cl.: A61N 1/372

(54) **CONTROL METHOD FOR PACING PULSE AMPLITUDE, AND CARDIAC IMPLANTABLE ELECTRONIC DEVICE**

(30) Priority: 12.10.2023 CN 202311322956
(71) Applicant: MicroPort Soaring CRM (Shanghai) Co., Ltd., Shanghai 201203 (CN)
(72) Inventor: WANG, Yulin, Shanghai 201203 (CN); LIAO, Wangcai, Shanghai 201203 (CN)
(74) Representative: Lavoix
(86) International application number: PCT/CN2024/120277
(87) International publication number: WO 2025/077552

(57) **Abstract**

A method for pacing pulse amplitude control and a cardiac implantable electronic device (CIED), the method is used in the CIED. An equivalent circuit of the CIED includes pacing discharge and charge circuits. The pacing charge circuit includes a charge optimization circuit including at least two parallel-connected charge optimization sub-circuits each including a resistor and a switch connected in series with the resistor. The resistances of the resistors in any two of the charge optimization sub-circuits are different. The method includes: when charging the first capacitor in the CIED, disconnecting the pacing discharge circuit and conducting at least one of the charge optimization sub-circuits with different resistance values depending on whether the CIED is in an MR interfering environment. This can reduce the risk of amplitude attenuation of pacing pulses caused by EMI in an MRI and ensures successful cardiac pacing of the CIED.

## Description

### TECHNICAL FIELD

The present invention relates to the field of medical devices and, in particular, to a method for pacing pulse amplitude control and a cardiac implantable electronic device (CIED).

### BACKGROUND

Magnetic resonance imaging (MRI), a non-invasive, ionizing radiation-free imaging technique, has been widely used in clinical diagnosis in cardiology, oncology and neurology. Recognized as the most effective, currently available medical devices for bradycardia treatment and sudden cardiac death (SCD) prevention, cardiac implantable electronic devices (CIEDs), represented by implantable cardiac pacemakers and implantable cardioverter-defibrillators (ICDs), are active devices suitable for long-term implantation in the human body. The increasingly widespread use of MRI diagnostics and CIEDs has brought about an increasing demand of patients with CIEDs for MRI scans. MRI systems utilize strong static magnetic fields, radio frequency (RF) fields and gradient fields to generate images of the human body. However, such a strong magnetic field could interfere with the functioning of a CIED, increasing the risk of damage to a patient with the CIED who is receiving an MRI scan. Possible consequences that can be expected include tissue heating, pacing inhibition, tachycardia induced by the device, displacement of the device, damaged components thereof, incorrect diagnostics, etc.

However, for life-supporting implantable medical devices such as an implantable cardiac pacemaker or an implantable defibrillator, even when a pacing-dependent patient is undergoing an MRI scan, the CIED must still continuously deliver pacing pulses at a predetermined frequency and amplitude to maintain the patient's normal heartbeat. If the waveform of such pacing pulses is interfered with by RF signals used in the MRI scan, the amplitude of the pulses may be attenuated to a level that is too low to allow the pulses to successfully pace the heart. For example, a resonance frequency of a 1.5 T MRI machine (operating at a Larmor frequency of 63.9 MHz) is 64 MHz, and a resonance frequency of a 3 T MRI machine (operating at a Larmor frequency of 127.8 MHz) is 128 MHz.

Therefore, how to reduce the risk of amplitude attenuation of pacing pulses caused by electromagnetic interference (EMI) in an MRI environment represents an increasingly urgent challenge in the art.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY OF THE INVENTION

It is an object of the present invention to provide a method for pacing pulse amplitude control and a cardiac implantable electronic device (CIED), which address possible failure of pacing pulses from a conventional CIED to pace the heart due to interference-induced amplitude attenuation when it is used in an MRI environment. A charge circuit for a first capacitor is optimized to lower the risk of amplitude attenuation of pacing pulses caused by EMI in an MRI, ensuring successful cardiac pacing of the CIED.

To this end, the present invention provides a method for pacing pulse amplitude control used in a CIED. An equivalent circuit of the CIED comprises contact impedance between an electrode lead and myocardial tissue, electrode resistance of the electrode lead, a filter, a first capacitor, a discharge switch and a power supply. The contact impedance, the electrode resistance, the filter, the first capacitor and the discharge switch are connected in series to form a discharge circuit, and the first capacitor and the power supply are connected in series and form a pacing charge circuit. The pacing charge circuit further comprises a charge optimization circuit connected in series with the power supply. The charge optimization circuit comprises at least two parallel-connected charge optimization sub-circuits each comprising a resistor and a switch connected in series with the resistor. The resistances of the resistors in any two of the charge optimization sub-circuits are different.

The method comprises, when charging the first capacitor, opening the discharge switch and performing the steps of:
if the CIED is determined not to be in a magnetic resonance (MR) interfering environment, conducting at least one of the charge optimization sub-circuits having a higher resistance, and disconnecting the other charge optimization sub-circuit(s); and
if the CIED is determined to be in an MR interfering environment, conducting at least one of the charge optimization sub-circuits having a lower resistance, and disconnecting the other charge optimization sub-circuit(s).

Optionally, the charge optimization circuit may comprises two parallel-connected charge optimization sub-circuits, wherein a first charge optimization sub-circuit comprises a first resistor and a first switch that are connected in series, wherein a second charge optimization sub-circuit comprises a second resistor and a second switch that are connected in series, wherein the first resistor has a higher resistance than the second resistor, wherein the method comprises, in case of charging the first capacitor, opening the discharge switch and performing the steps of:
if the CIED is determined not to be in an MR interfering environment, closing the first switch and opening the second switch, thereby forming the pacing charge circuit that is in a conductive state by the power supply, the first switch, the first resistor and the first capacitor, wherein the power supply charges the first capacitor through the first resistor; and
if the CIED is determined to be in an MR interfering environment, closing the second switch and opening the first switch, thereby forming the pacing charge circuit that is in a conductive state by the power supply, the second switch, the second resistor and the first capacitor, wherein the power supply charges the first capacitor through the second resistor.

Optionally, the resistance of the first resistor may be in the range of 4 MΩ to 6 MΩ, and the resistance of the second resistor may be in the range of 100 Ω to 200 Ω.

Optionally, a first terminal of the charge optimization circuit may be connected to both a first terminal of the filter and a first terminal of first capacitor, with a second terminal of the charge optimization circuit being electrically connected to a positive terminal of the power supply.

Optionally, the method may further comprise, subsequent to the charging of the first capacitor, conducting the pacing discharge circuit and disconnecting the pacing charge circuit, thereby allowing the first capacitor to deliver a pacing pulse.

Optionally, the filter may comprise a second capacitor, a discharge optimization circuit and an inductor, wherein the second capacitor and the discharge optimization circuit are connected in series and then are connected in parallel to the inductor, wherein the discharge optimization circuit comprises at least two parallel-connected discharge optimization sub-circuits, wherein each discharge optimization sub-circuit comprises a resistor and a switch that are connected in series, wherein resistances of the resistors in any two of the discharge optimization sub-circuits are different, and

wherein the method comprises, when the first capacitor is in a non-charging state, disconnecting the charge optimization circuit and performing the steps of:
if the CIED is determined not to be in an MR interfering environment, disconnecting the discharge optimization circuit and closing a discharge switch, wherein the first capacitor delivers a pacing pulse through the inductor and the second capacitor;
if the CIED is determined to be in an MR interfering environment and a pacing pulse delivery is not needed, conducting at least one of the discharge optimization sub-circuits having a higher resistance and opening the discharge switch and the other discharge optimization sub-circuit(s), wherein the second capacitor confines radio frequency of an interfering signal source within the filter through the conducted discharge optimization sub-circuit and the inductor; and
if the CIED is determined to be in an MR interfering environment and a pacing pulse delivery is needed, conducting at least one of the discharge optimization sub-circuits having a lower resistance and the discharge switch, and disconnecting the other discharge optimization sub-circuits wherein the first capacitor delivers a pacing pulse through the conducted discharge optimization sub-circuit and the second capacitor.

Optionally, the discharge optimization circuit may comprise two parallel-connected discharge optimization sub-circuits, wherein a first discharge optimization sub-circuit comprises a third resistor and a third switch that are connected in series, wherein a second discharge optimization sub-circuit comprises a fourth resistor and a fourth switch that are connected in series, wherein the third resistor has a higher resistance than the fourth resistor, and
wherein the method comprises, when the first capacitor is in a non-charging state, disconnecting the charge optimization circuit and performing the steps of:
if the CIED is determined not to be in an MR interfering environment, opening the third and fourth switches and closing the discharge switch, thereby forming the pacing discharge circuit that is in a conductive state by the first capacitor, the inductor, the second capacitor, the electrode resistance, the contact impedance and the discharge switch, wherein the first capacitor delivers a pacing pulse through the inductor and the second capacitor;
if the CIED is determined to be in an MR interfering environment and a pacing pulse delivery is not needed, closing the third switch and opening the fourth and discharge switches, thereby forming the pacing discharge circuit that is in a conductive state by the second capacitor, the inductor, the third switch and the third resistor, wherein the second capacitor confines radio frequency of the interfering signal source within the filter through the third resistor and the inductor; and
if the CIED is determined to be in an MR interfering environment and a pacing pulse delivery is needed, closing the fourth and discharge switches and opening the third switch, thereby forming the pacing discharge circuit that is in a conductive state by the first capacitor, the inductor, the fourth switch, the fourth resistor, the second capacitor, the electrode resistance, the contact impedance and the discharge switch, wherein the first capacitor delivers a pacing pulse through the fourth resistor and the second capacitor.

Optionally, the resistance of the third resistor may be in the range of 1 MΩ to 5 MΩ, and the resistance of the fourth resistor may be in the range of 100 Ω to 500 Ω.

Optionally, a first terminal of the discharge optimization circuit may be connected to each of a first terminal of the inductor, a first terminal of the charge optimization circuit and a first terminal of first capacitor, wherein a second terminal of the discharge optimization circuit is connected to a first terminal of the second capacitor, and a second terminal of the inductor is connected between the electrode resistance and the second capacitor.

Optionally, the filter may be any of a band-stop filter, a passive filter, an active filter and a digital filter.

Optionally, a resonant frequency of the filter may be equal to an MRI radio frequency.

To the above end, the present invention also provides a CIED, which uses the method as defined above for pacing pulse amplitude control.

Optionally, the CIED may be implemented as an implantable cardiac pacemaker, or as an implantable cardioverter-defibrillator (ICD).

To the above end, the present invention further provides a CIED comprising a contact impedance between an electrode lead and myocardial tissue, an electrode resistance of the electrode lead, a filter, a first capacitor, a discharge switch and a power supply, wherein the contact impedance, the electrode resistance, the filter, the first capacitor and the discharge switch are connected in series and form a pacing discharge circuit, wherein the first capacitor and the power supply are connected in series and form a pacing charge circuit, wherein the pacing charge circuit further comprises a control unit and a charge optimization circuit connected in series with the power supply, wherein the charge optimization circuit comprises at least two parallel-connected charge optimization sub-circuits, wherein each charge optimization sub-circuit comprises a resistor and a switch that are connected in series, wherein resistances of the resistors in any two of the charge optimization sub-circuits are different, wherein the control unit is configured to control pacing pulse amplitude depending on whether the CIED is in an MR interfering environment, thereby avoiding or reducing MR interference.

Optionally, the control unit may be adapted to implement the method for pacing pulse amplitude control as defined above.

The present invention has the following benefits over the prior art:
It provides a method for pacing pulse amplitude control used in a CIED. A pacing charge circuit of the CIED includes a charge optimization circuit, which includes at least two parallel-connected charge optimization sub-circuits each including a resistor and a switch connected in series with the resistor. The resistances of the resistors in any two of the charge optimization sub-circuits are different. The method includes, in case of charging the first capacitor, opening the discharge switch and performing the steps of: if the CIED is determined not to be in a magnetic resonance (MR) interfering environment, conducting at least one of the charge optimization sub-circuits having a higher resistance, and disconnecting the other charge optimization sub-circuit(s); and if the CIED is determined to be in an MR interfering environment, conducting at least one of the charge optimization sub-circuits having a lower resistance, and disconnecting the other charge optimization sub-circuit(s). Therefore, the method of the present invention fully leverages the fact that the time constant of an RC charge circuit (e.g., consisting of a resistor in the charge optimization circuit and the first capacitor) is proportional to the product of the resistance and capacitance, and charging the first capacitor through a smaller resistor when in an MR interfering environment allowing the charging to be completed within a much shorter time and hence rendering the first capacitor more resistant to EMI in the MRI. As a result, during the pacing discharge of the cardiac implantable electronic device (specifically the first capacitor), the risk of a decrease in pacing pulse amplitude caused by an EMI in MRI is reduced and the health of a patient during an MRI check can better protected. In addition, the risk of incorrect diagnostics associated with attenuated pacing pulses from the CIED can be reduced, and successful cardiac pacing of the CIED can be ensured.

In addition, in the method for pacing pulse amplitude control of the present invention, a filter may include a second capacitor, a discharge optimization circuit and an inductor. The second capacitor and the discharge optimization circuit may be connected in series with each other and then together in parallel to the inductor. The discharge optimization circuit may include at least two parallel-connected discharge optimization circuits each including a resistor and a switch connected in series with the resistor. The resistances of the resistors in any two of the charge optimization sub-circuits are different. In this case, the method may include, when charging the first capacitor, disconnecting the charge optimization circuit, closing the discharge switch and performing the steps of: if it is determined that the CIED is not in an MR interfering environment, disconnecting the discharge optimization circuit; if it is determined that the CIED is in an MR interfering environment and that there is no need for a pacing pulse delivery, conducting at least one of the discharge optimization sub-circuits, whichever has higher resistance, and disconnecting the other discharge optimization sub-circuit(s); and if it is determined that the CIED is in an MR interfering environment and that there is a need for a pacing pulse delivery, conducting at least one of the discharge optimization sub-circuits, whichever has lower resistance, and the discharge switch, and disconnecting the other discharge optimization sub-circuit(s). Therefore, in the method for pacing pulse amplitude control of the present invention, the discharge optimization circuit added to the pacing discharge circuit is of a novel design, which can provide appropriate quality factors Q for various scenarios by changing different resistors connected into a band-stop LC filter through controlling the states of associated switches. This addresses the contradiction between filtering effect and an impact on the amplitude of pacing pulses in an MR radio frequency filed and allows pacing pulses to be delivered to the heart almost without being affected.

It also provides a CIED, which embodies the same inventive concept as the method for pacing pulse amplitude control and therefore has at least all of its advantages. For more details of these advantages, reference is made to the above description in connection with the method for pacing pulse amplitude control, and further description thereof is omitted for the sake of brevity and conciseness.

### BRIEF DESCRIPTION OF THE DRAWINGS

Fig. 1 shows a schematic flowchart of a method for pacing pulse amplitude control according to a first embodiment of the present invention.
Fig. 2 shows a simplified equivalent circuit diagram of a specific example of a cardiac implantable electronic device (CIED), in which the method according to the first embodiment of the present invention can be employed.
Fig. 3 shows a simplified equivalent circuit diagram of the CIED of Fig. 2 during its use in a magnetic resonance (MR) interfering environment.
Fig. 4 shows a schematic flowchart of a method for pacing pulse amplitude control according to a second embodiment of the present invention.
Fig. 5 shows a simplified equivalent circuit diagram of a specific example of a CIED, in which the method according to the second embodiment of the present invention can be employed.
Fig. 6 shows a simplified equivalent circuit diagram of the CIED of Fig. 5 during its use in an MR interfering environment.
Fig. 7 schematically illustrates how the use of different discharge optimization circuits in the CIED of Fig. 6 impacts a pacing pulse in a 64-MHz MR environment.
Fig. 8 schematically illustrates how the use of different discharge optimization circuits in the CIED of Fig. 6 impacts a pacing pulse in a 128-MHz MR environment.

### List of Reference Numerals

first resistor-R1; second resistor-R2; third resistor-R3; fourth resistor-R4; electrode resistance-R5; contact impedance-R6;
first switch-K1; second switch-K2; third switch-K3; fourth switch K4; discharge switch-K5;
filter-LC; first capacitor-C1; power supply-E1; interfering signal source-Emri; second capacitor-C2; inductor- L;
pacing charge circuit-200; charge optimization circuit-210; pacing discharge circuit -100; discharge optimization circuit-110.

### DETAILED DESCRIPTION

Objects, advantages and features of the present invention will become more apparent upon reading the following more detailed description of methods for pacing pulse amplitude control and cardiac implantable electronic devices (CIEDs) described herein with reference to the accompanying drawings. Note that the figures are provided in a very simplified form not necessarily drawn to exact scale for the only purpose of helping to explain the disclosed embodiments in a more convenient and clearer way. It will be understood that the figures may not necessarily illustrate the structures described herein to scale and that illustrative features depicted in the figures to explain certain principles of the present invention may be slightly simplified. The specific design features of the invention as disclosed herein, including, for example, specific dimensions, orientations, locations and shapes, will be determined in part by the particular intended application and use environment. Additionally, in the embodiments described below, same reference numerals may be sometimes used to refer to the same or functionally same elements throughout different figures, while description thereof may not be repeated. In this specification, same reference numerals and letters refer to same items in the figures, and thus once an item is defined in one figure, it may not be discussed for following figures.

Where appropriate, the terms so used are interchangeable. Further, if a method is described herein as comprising a series of steps, the order of these steps as presented herein is not necessarily the only order in which they can be performed, and some of the stated steps may be omitted and/or other steps not described herein may be added to the method.

In addition, as used herein, the terms "first", "second" and the like are intended only for illustration and are not to be construed as denoting or implying relative importance, or as implicitly indicating any particular number of the referenced items. Accordingly, defining an item with "first", "second" or the like is an explicit or implicit indication of the presence of at least one such item. As used herein, the term "plurality" means "at least two", such as two or three, unless otherwise clearly defined.

Before describing method for pacing pulse amplitude control disclosed herein in detail, it is to be noted that for ease of understanding and description of the present invention, the description of Embodiment 1 below focuses on the optimization of a pacing charge circuit for a pacing capacitor (also referred to hereinafter as a first capacitor) according to a method for pacing pulse amplitude control described herein, while the description of Embodiment 2 below focuses on the optimization of a pacing discharge circuit for a pacing capacitor (also referred to hereinafter as a first capacitor) according to a method for pacing pulse amplitude control described herein. It will be understood that the pacing charge circuit optimization scheme and the pacing discharge circuit optimization scheme according to the methods described herein are not contradictory to, or exclusive of, each other at all, but can be used either separately or in combination. Specifically, in applications of methods for pacing pulse amplitude control according to the present invention, either or both of the pacing charge circuit optimization scheme described in Embodiment 1 and the pacing discharge circuit optimization scheme described in Example 2 may be employed. That is, the two schemes may be used simultaneously to achieve optimization of both pacing charge and discharge circuits.

### EMBODIMENT 1

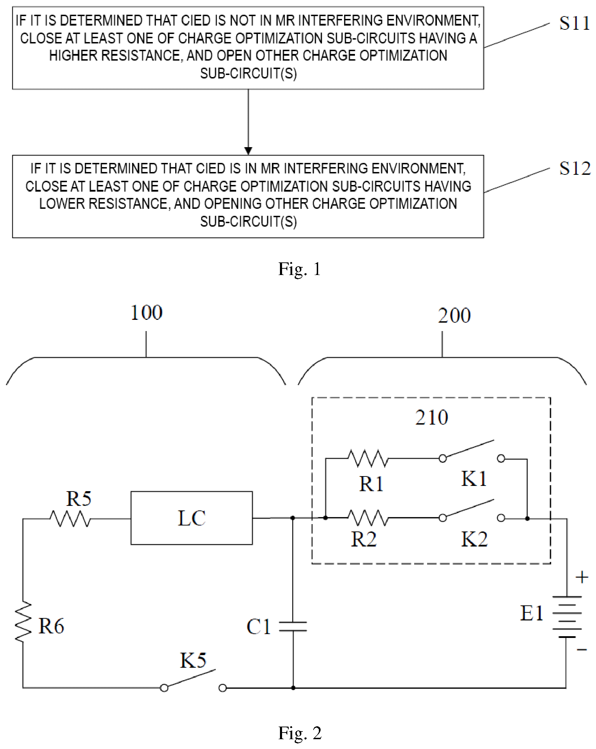

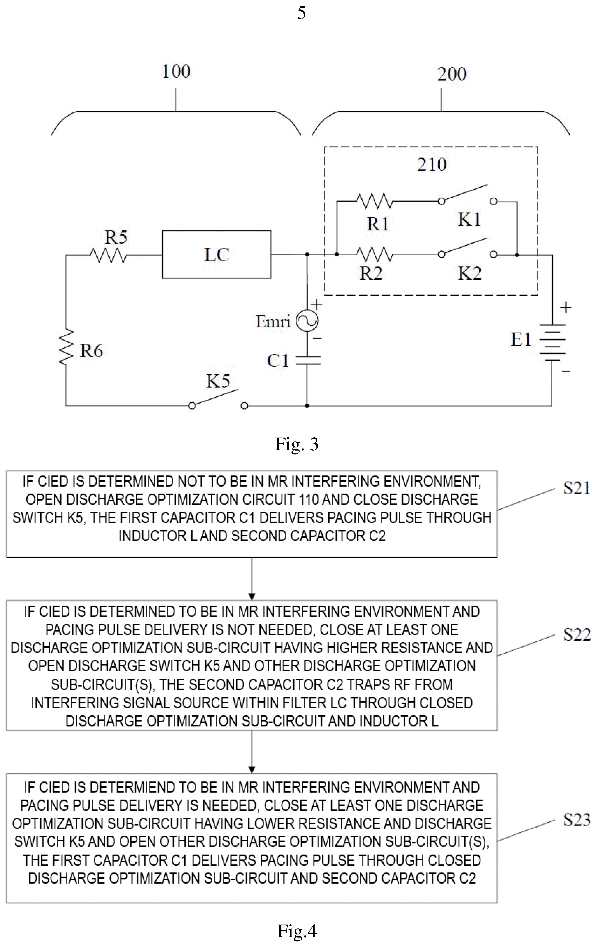

In a first embodiment of the present invention, there is provided a method for pacing pulse amplitude control in a cardiac implantable electronic device (CIED). Reference is now made to Figs. 1, 2 and 3. Fig. 1 shows a schematic flowchart of the method for pacing pulse amplitude control according to the first embodiment. Fig. 2 shows a simplified equivalent circuit diagram of a specific example of the CIED in the method for pacing pulse amplitude control of the first embodiment. Fig. 3 shows a simplified equivalent circuit diagram of the CIED of Fig. 2 during its use in an MR interfering environment. As can be seen from Figs. 1, 2 and 3, the equivalent circuits of the CIED include contact impedance R6 between an electrode lead and myocardial tissue, electrode resistance R5 of the electrode lead, a filter LC, a first capacitor C1, a discharge switch K5 and a power supply E1. The contact impedance R6, the electrode resistance R5, the filter LC, the first capacitor C1 and the discharge switch K5 are connected in series to form a pacing discharge circuit 100, and the first capacitor C1 and the power supply E1 are connected in series to form a pacing charge circuit 200. The pacing charge circuit 200 further includes a charge optimization circuit 210 connected in series with the power supply E1. The charge optimization circuit 210 includes at least two parallel-connected charge optimization sub-circuits (not labeled) each including a resistor and a switch connected in series with the resistor. The resistances of the resistors in any two of the charge optimization sub-circuits are different.

The method includes, in case of charging the first capacitor, opening the discharge switch and performing the steps of:
S11: if it is determined that the CIED is not in an MR interfering environment, conducting at least one of the charge optimization sub-circuits, whichever has higher resistance, and disconnecting the other charge optimization sub-circuit(s); and
S12: if it is determined that the CIED is in an MR interfering environment, conducting at least one of the charge optimization sub-circuits, whichever has lower resistance, and disconnecting the other charge optimization sub-circuit(s).

Therefore, the method for pacing pulse amplitude control of this embodiment fully leverages the fact that the time constant of an RC charge circuit (e.g., consisting of a resistor in the charge optimization circuit 210 and the first capacitor C1) is proportional to the product of the resistance and capacitance to charge the first capacitor C1 through a smaller resistor when in an MR interfering environment, allowing the charging to be completed within a much shorter time and hence rendering the first capacitor C1 more resistant to electromagnetic interference (EMI) in the MRI. As a result, during the pacing discharge of the cardiac implantable electronic device (specifically, the first capacitor C1), the risk of a decrease in pacing pulse amplitude caused by an EMI in MRI is reduced and the health of a patient during an MRI check can better protected. In addition, the risk of incorrect diagnostics associated with attenuated pacing pulses from the CIED can be reduced, and successful cardiac pacing of the CIED can be ensured.

It should be noted, and will be appreciated by those skilled in the art, that the schematic equivalent circuit diagrams of the CIED shown in Figs. 2 and 3 are merely for ease of description and understanding and not intended to limit the present invention in any sense. In other words, although the charge optimization circuit 210 has been illustrated in Figs. 2 and 3 as having two parallel-connected charge optimization sub-circuits, the present embodiment is not so limited, as in alternative embodiments not shown for the sake of brevity, the charge optimization circuit 210 may include a different number of charge optimization sub-circuits, such as three, four or more charge optimization sub-circuits.

Further, the present invention is not limited to any particular value of the contact impedance R6 between the electrode lead and the myocardial tissue or of the electrode resistance R5 of the electrode lead, and such values may depend on considerations in practical applications. For example, the contact impedance R6 may be about 1 kΩ, and the electrode resistance R5 of the electrode lead may be about 50 Ω.

In particular, this embodiment is not limited to any particular type of filter LC, and the filter LC may be implemented as any of a band-stop filter, a passive filter, an active filter and a digital filter. Additionally, as shown in Fig. 3, when the CIED is used in an MR interfering environment, the present invention is not limited to any particular interfering signal source Emri. For example, a radio frequency (RF) of the interfering signal source Emri may be 64 MHz (corresponding to a 1.5 T MRI environment) or 128 MHz (corresponding to a 3 T MRI environment). Of course, while not described in detail herein, the radio frequency of the interfering signal source Emri may be any other frequency than 64 MHz and 128 MHz.

Further, those skilled in the art will understand that the present invention is not limited to any particular order in which steps S11 and S12 are performed. Although these steps have been described above in the order of S11 -> S12, apparently, this is merely a matter of wording and not intended to limit the present invention in any sense. In fact, as the first capacitor C1 is charged as the CIED is being either in an MR interfering environment, or not, it is indeed unnecessary for steps S11 and S12 to be performed in any particular order.

For ease of understanding and explanation of the present invention, the method is further described below in the context of the charge optimization circuit 210 including two parallel-connected charge optimization sub-circuits, as an example.

With continued reference to Figs. 2 and 3, in this embodiment, the charge optimization circuit 210 may include two parallel-connected charge optimization sub-circuits, one of which includes a first resistor R1 and a first switch K1 connected in series with the first resistor R1, and the other includes a second resistor R2 and a second switch K2 connected in series with the second resistor R2. The resistance of the first resistor R1 is higher than that of the second resistor R2. In this case, the method includes, when charging the first capacitor C1, opening the discharge switch K5 and performing the steps of:
if it is determined that the CIED is not in an MR interfering environment, closing the first switch K1 and opening the second switch K2, thereby forming the pacing charge circuit 200 that is in a conductive state by the power supply E1, the first switch K1, the first resistor R1 and the first capacitor C1,and the power supply E1 charges the first capacitor C1 through the first resistor R1; and
if it is determined that the CIED is in an MR interfering environment, closing the second switch K2 and opening the first switch K1, thereby forming the pacing charge circuit 200 that is in a conductive state by the power supply E1, the second switch K2, the second resistor R2 and the first capacitor C1, and the power supply E1 charges the first capacitor C1 through the second resistor R2.

Thus, in the method for pacing pulse amplitude control of this embodiment, in contrast to the first capacitor C1 being charged by the power supply E1 through the larger first resistor R1 when the CIED is not in an MR interfering environment, the first capacitor C1 is charged by the power supply E1 through the smaller second resistor R2 when the CIED is in an MR interfering environment. This allows the charging of the first capacitor C1 to be completed within a much shorter time, thereby rendering the first capacitor C1 more resistant to EMI in the MRI. As a result, during the pacing discharge of the cardiac implantable electronic device (specifically, the first capacitor C1), the risk of a decrease in pacing pulse amplitude caused by an EMI in MRI is reduced and the health of a patient during an MRI check can better protected. In addition, the risk of incorrect diagnostics associated with attenuated pacing pulses from the CIED can be reduced, and successful cardiac pacing of the CIED can be ensured.

In particular, in this embodiment, the resistance of the first resistor R1 may be 5 MΩ, and the resistance of the second resistor R2 may be 150 Ω. However, the present invention is not limited to any particular resistance value of the resistor in each charge optimization sub-circuit, as in alternative embodiments, the resistance of the first resistor R1 may vary in the range of 4 MΩ to 6 MΩ, and the resistance of the second resistor R2 may vary in the range of 100 Ω to 200 Ω, depending on the severity of interference in an MRI environment where the CIED is intended to be used. This allows the CIED to have different charge time depending on a radio frequency of an interfering signal source Emri. As a result, the EMI resistance of the first capacitor C1 can be adapted to address a broader range of applications.

Accordingly, in the method for pacing pulse amplitude control of this embodiment, since the resistance of the second resistor R2 may be much lower than that of the first resistor R1, according to an RC charge circuit time constant is proportional to the product of the resistance and capacitance, when the first capacitor C1 is charged through the second resistor R2, the charging time can be significantly reduced, rendering the first capacitor C1 more resistant to EMI in an MRI. As a result, during the pacing discharge of the cardiac implantable electronic device (specifically, the first capacitor C1), the risk of a decrease in pacing pulse amplitude caused by an EMI in MRI is reduced and the health of a patient during an MRI check can better protected. In addition, the risk of incorrect diagnostics associated with attenuated pacing pulses from the CIED can be reduced, and successful cardiac pacing of the CIED can be ensured.

In some preferred exemplary implementations, a first terminal of the charge optimization circuit 210 is connected to both a first terminal of the filter LC and a first terminal of the first capacitor C1, and a second terminal of the charge optimization circuit 210 is electrically connected to a positive terminal of the power supply E1. With this arrangement, the charge optimization circuit 210 used in the method for pacing pulse amplitude control of this embodiment can be connected in a straightforward way in the circuit of the CIED, making it easier to implement and maintain.

Once charging of the first capacitor C1 is completed, the first capacitor C1 may be discharged to deliver a pacing pulse. Accordingly, the method for pacing pulse amplitude control further includes, subsequent to the charging of the first capacitor C1, conducting the pacing discharge circuit 100 and disconnecting the pacing charge circuit 200, thereby allowing the first capacitor C1 to deliver a pacing pulse. Thus, in the method for pacing pulse amplitude control of this embodiment, the first capacitor C1 is charged by the pacing charge circuit 200, and the pacing discharge circuit 100 is then conducted to cause the first capacitor C1 to deliver a pacing pulse. Since the charging is completed before the pulse is delivered, the CIED can successfully pace the heart, whether it is in an MR interfering environment, or not.

Specifically, as would be appreciated by those skilled in the art, in the method for pacing pulse amplitude control of this embodiment, the CIED may operate to pace the heart successfully by repeating a cycle of charging the first capacitor C1 by the pacing charge circuit 200 and then causing the first capacitor C1 to deliver a pacing pulse by the pacing discharge circuit 100.

### EMBODIMENT 2

For ease of understanding of a method for pacing pulse amplitude control according to a second embodiment, before describing it in detail, research involved in its development and its basic principles are first briefed below.

In order to enable safer use of a CIED in an MRI environment, numerous improvements have been proposed in the related art, which typically involve the use of a band-stop filter LC for limiting the propagation of magnetic field frequency in the MRI environment, enhancing the shunting and dissipation of magnetically induced currents, or shielding the pulse generator in a titanium or stainless steel enclosure, so as to avoid damage to its circuits.

As suggested by research, for a filter LC constructed of an inductor and a capacitor, which are connected in parallel, a desired quality factor Q can be achieved by carefully choosing a resistance value for a resistor in the filter LC, which enables the filter to filter out undesired MRI radio frequency while not affecting pacing pulses as much possible. However, these two goals are inherently incompatible - filtering out undesired MRI radio frequency requires sufficiently high resistance to ensure that the filter LC has a sufficient filtering bandwidth; however, a larger bandwidth inevitably means a greater adverse impact on pacing pulses, which may lead to the amplitude of the pulses being attenuated to a level that is too low to allow the pulses to successfully pace the heart. Therefore, the risk of amplitude attenuation of pacing pulses caused by EMI in an MRI cannot be effectively lowered, and a good tradeoff between the two goals cannot be found, simply by choosing an appropriate resistance value for the resistor in the filter LC (which determines the quality factor Q of the filter LC - a higher resistance value means a lower Q value, a broader filtering bandwidth, lower filtering efficiency and a greater adverse impact on pacing pulses; and vice versa). Therefore, the problem of amplitude attenuation of pacing pulses cannot be satisfactorily solved in this way.

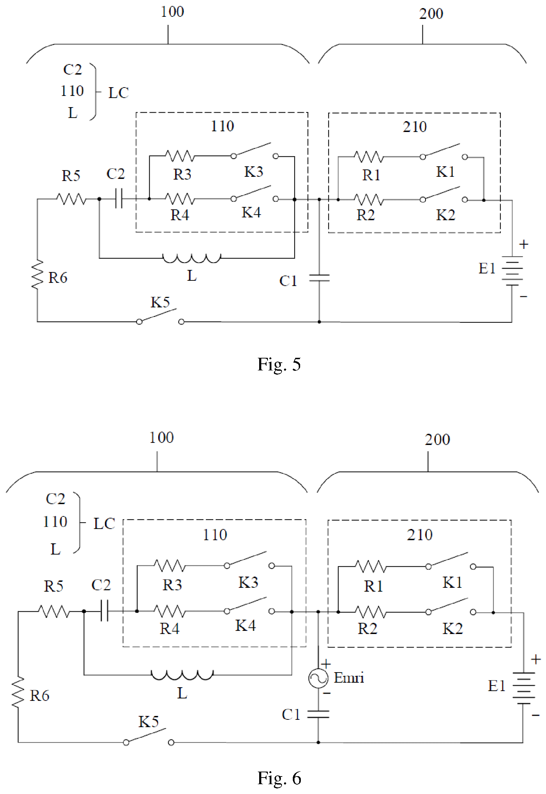

In view of the above, this embodiment provides another method for pacing pulse amplitude control. Differing from the method of the first embodiment, in which the pacing charge circuit 200 is optimized, a pacing discharge circuit 100 is optimized in the method for pacing pulse amplitude control of this embodiment. Reference is now made to Figs. 4, 5 and 6. Fig. 4 shows a schematic flowchart of the method according to the second embodiment. Fig. 5 shows a simplified equivalent circuit diagram of a specific example of a CIED in the method for pacing pulse amplitude control of the second embodiment. Fig. 6 shows a simplified equivalent circuit diagram of the CIED of Fig. 5 during its use in an MR interfering environment. As shown in Figs. 4, 5 and 6, in the method for pacing pulse amplitude control of this embodiment, the CIED includes a filter LC including a second capacitor C2, a discharge optimization circuit 110 and an inductor L. The second capacitor C2 and the discharge optimization circuit 110 are connected in series and then together in parallel to the inductor L. The discharge optimization circuit 110 includes at least two parallel-connected discharge optimization sub-circuits (not labeled) each including a resistor and a switch connected in series with the resistor. The resistances of the resistors in any two of the charge optimization sub-circuits are different. The method includes, when a first capacitor C1 is in a non-charged, disconnecting the charge optimization circuit 210 and performing the steps of:
S21: if it is determined that the CIED is not in an MR interfering environment, disconnecting the discharge optimization circuit 110 and closing a discharge switch K5, and the first capacitor C1 delivers a pacing pulse through the inductor L and the second capacitor C2;
S22: if it is determined that the CIED is in an MR interfering environment and that there is no need for a pacing pulse delivery, conducting at least one of the discharge optimization sub-circuits, whichever has higher resistance and opening the discharge switch K5 and the other discharge optimization sub-circuit(s), and the second capacitor C2 confines a radio frequency of an interfering signal source within the filter LC through the conducted discharge optimization sub-circuit and the inductor L; and
S23: if it is determined that the CIED is in an MR interfering environment and that there is a need for a pacing pulse delivery, conducting at least one of the discharge optimization circuits, whichever has lower resistance, and the discharge switch K5 and disconnecting the other discharge optimization circuit(s), and the first capacitor C1 delivers a pacing pulse through the conducted discharge optimization sub-circuit and the second capacitor C2.

In the method for pacing pulse amplitude control of this embodiment, the discharge optimization circuit 110 added to the pacing discharge circuit 100 is of a novel design, which can provide appropriate quality factors Q for various scenarios by changing resistors connected into the filter LC (which is essentially a band-stop LC filter) through controlling the states of different switches. This addresses the contradiction between filtering effect of MR radio frequency and an impact on the amplitude of pacing pulses and allows pacing pulses to be delivered to the heart almost without being compromised at all.

It should be noted, and will be appreciated by those skilled in the art, that the schematic equivalent circuit diagrams of the CIED shown in Figs. 5 and 6 are merely for ease of description and understanding and not intended to limit the present invention in any sense. In other words, although the discharge optimization circuit 110 has been illustrated in Figs. 5 and 6 as having two parallel-connected discharge optimization sub-circuits, the present embodiment is not so limited, as in alternative embodiments not shown for the sake of brevity, the discharge optimization circuit 110 may include a different number of discharge optimization sub-circuits, such as three, four or more charge optimization sub-circuits.

Further, those skilled in the art will understand that the present invention is not limited to any particular order in which steps S21, S22 and S23 are performed. Although these steps have been described above in the order of S21 -> S22 -> S23, apparently, this is merely a matter of limitation of wording and not intended to limit the present invention in any sense. In fact, as the CIED is either not in an MR interfering environment, or in an MR interfering environment with or without a need for a pacing pulse delivery, it is indeed unnecessary for steps S21, S22 and S23 to be performed in any particular order.

For ease of understanding and explanation of the present invention, the method is further described below in the context of the discharge optimization circuit 110 including two parallel-connected discharge optimization sub-circuits, as an example.

With continued reference to Figs. 5 and 6, in this embodiment, the discharge optimization circuit 110 may include two parallel-connected discharge optimization sub-circuits, one of which includes a third resistor R3 and a third switch K3 connected in series with the third resistor R3, and the other includes a fourth resistor R4 and a fourth switch K4 connected in series with the fourth resistor R4. The resistance of the third resistor R3 is higher than that of the fourth resistor R4.

In this case, the method includes, when the first capacitor C1 is not being charged, disconnecting the charge optimization circuit and performing the steps described below.

If it is determined that the CIED is not in an MR interfering environment, the third and fourth switches K3, K4 are opened and the discharge switch K5 is closed, thereby forming the pacing discharge circuit 100 that is in a conductive state by the first capacitor C1, the inductor L, the second capacitor C2, electrode resistance R5, contact impedance R6 and the discharge switch K5, and the first capacitor C1 delivers a pacing pulse through the inductor L and the second capacitor C2.

Specifically, when the CIED is not in an MR environment, the third and fourth switches K3, K4 may be opened. A low-frequency pacing pulse from the first capacitor C1 propagates through the inductor L and acts on the heart almost without being affected, thereby achieving the pacing.

If it is determined that the CIED is in an MR interfering environment and that there is no need for a pacing pulse delivery, the third switch K3 is closed and the fourth and discharge switches K4, K5 are opened, thereby forming the pacing discharge circuit 100 that is in a conductive state by the second capacitor C2, the inductor L, the third switch K3 and the third resistor R3, the second capacitor C2 confines a radio frequency of an interfering signal source within the filter LC through the third resistor R3 and the inductor L.

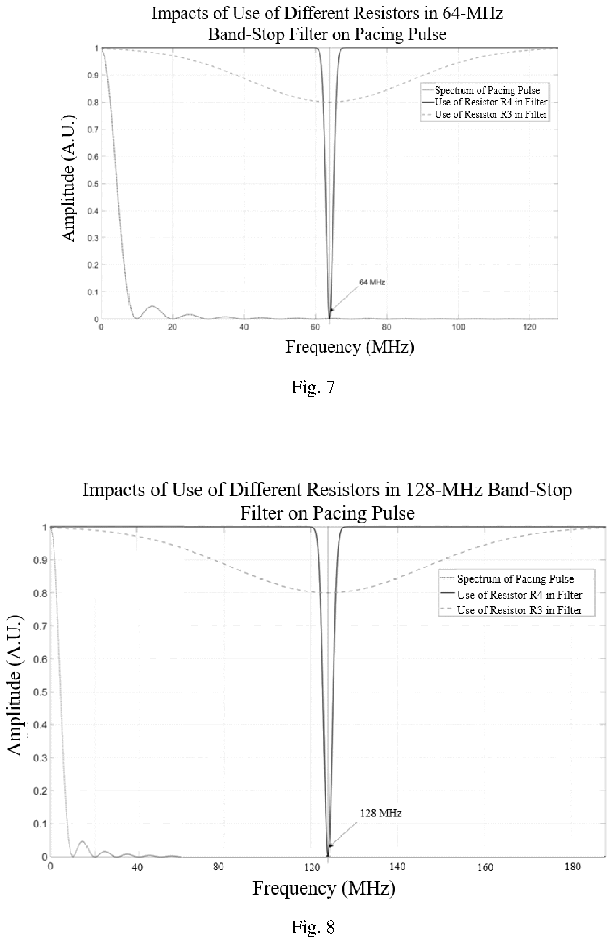

Reference is further made to Figs. 7 and 8. Fig. 7 schematically illustrates how the use of different discharge optimization sub-circuits in the CIED of Fig. 6 impacts a pacing pulse in a 64-MHz MR environment. Fig. 8 schematically illustrates how the use of different discharge optimization sub-circuits in the CIED of Fig. 6 impacts a pacing pulse in a 128-MHz MR environment. When the CIED is in an MR environment, if there is no need for a pacing pulse delivery, the third switch K3 is closed and the fourth switch K4 is opened, the resistor connected into the band-stop filter LC is the third resistor R3 having higher resistance. As a result, the band-stop filter LC has a lower Q value and a wider rejection bandwidth due to the higher resistance, enabling trapping of 64 or 128-MHz noise in the MR environment within the band-stop filter LC as much as possible. As such, an adverse impact that may otherwise exert on the CIED can be minimized, and an unexpected stimulus to the heart can be prevented.

It should be noted that, in this case, if a pacing pulse is delivered from the CIED, an upper bound of its frequency spectrum will have an overlap with a lower bound of the rejection band of the band-stop filter LC, leading to partial trapping of its energy within the band-stop filter LC. Consequently, when pacing pulse reaches an electrode of an electrode lead, its amplitude will be less than a preset value.

In particular, a resonant frequency of the filter LC is preferably equal to a radio frequency of the MRI, e.g., 64 MHz (corresponding to a 1.5 T MR environment) or 128 MHz (corresponding to a 3 T MR environment). The relationship between the resonant frequency and the filter's inductance and capacitance is: $f = \frac{1}{2 π \sqrt{LC}}$ wherein *f* is the resonant frequency of the filter LC, *L* is the inductance of the inductor L, and *C* is the capacitance of the second capacitor C2.

If it is determined that the CIED is in an MR interfering environment and that there is a need for a pacing pulse delivery, the fourth and discharge switches K4, K5 are closed and the third switch K3 is opened, thereby forming the pacing discharge circuit 100 that is in a conductive state by the first capacitor C1, the inductor L, the fourth switch K4, the fourth resistor R4, the second capacitor C2, the electrode resistance R5, the contact impedance R6 and the discharge switch K5, the first capacitor C1 delivers a pacing pulse through the fourth resistor R4 and the second capacitor C2.

When the CIED is used in an MR environment, if there is a need for a pacing pulse delivery, the third switch K3 is opened and the fourth switch K4 is closed, as a result, a fourth resistor R4 is connected into the band-stop filter LC. The band-stop filter LC has a higher Q value and a narrower rejection bandwidth due to the lower resistance. The band-stop filter LC confines most energy of 64 or 128-MHz noise in the MR environment therein while avoiding overlap between the rejection band of the band-stop filter LC and the frequency spectrum of the pacing pulse, as shown in Figs. 7 and 8. As such, the pacing pulse can reach the heart almost without being affected.

In this embodiment, the resistance of the third resistor R3 may be 3 MΩ, and the resistance of the fourth resistor R4 may be 300 Ω. However, the present invention is not limited to any particular resistance value of the resistor in each discharge optimization sub-circuit, as in alternative embodiments, the resistance of the third resistor R3 may vary in the range of 1 MΩ to 5 MΩ, and the resistance of the fourth resistor R4 may vary in the range of 100 Ω to 500 Ω, depending on the severity of interference in an MR environment where the CIED is intended to be used. This still enables the filter LC in the CIED to confine most energy of radio frequency (noise) of an interfering signal source Emri, allowing pacing pulses to reach the heart almost without being affected.

Therefore, the resistance of the third resistor R3 can impart desirable filtering capabilities while ensuring a sufficient filtering bandwidth. Moreover, it can be chosen without taking into account its impact on pacing pulses. The resistance of the fourth resistor R4 enables the delivery of pacing pulses with amplitude that suffices to capture the myocardium, while ensuring that 64 MHz (a frequency of RF signals in a 1.5 T MR environment) and 128 MHz (a frequency of RF signals in a 1.5 T MR environment) are covered. Thus, damage to the human body and the CIED can be reduced.

Specifically, in this embodiment, a first terminal of the discharge optimization circuit 110 is connected to each of a first terminal of the inductor L, a first terminal of the charge optimization circuit 210 and a first terminal of the first capacitor C1, and a second terminal of the discharge optimization circuit 110 is connected to a first terminal of the second capacitor C2, with a second terminal of the inductor L being connected between electrode resistance R5 and the second capacitor C2. With this arrangement, the discharge optimization circuit 110 used in the method for pacing pulse amplitude control of this embodiment can be connected in a straightforward way in the circuit of the CIED, making it easier to implement and maintain.

It should be noted that the method for pacing pulse amplitude control of this embodiment can be used in combination with the method of the first embodiment to provide even better pacing pulse amplitude control.

### EMBODIMENT 3

In a third embodiment, there is provided a CIED, in which the method for pacing pulse amplitude control of any of the foregoing embodiments can be employed for pacing pulse amplitude control. Since the CIED of this embodiment embodies the same inventive concept as the methods for pacing pulse amplitude control of the above embodiments, it has at least all the advantages of the methods for pacing pulse amplitude control. For more details of these advantages, reference is made to the above description in connection with the methods, and further description thereof is omitted for the sake of brevity and conciseness. In addition, a control unit implemented in software, hardware or both may be used to perform either or both of the methods for pacing pulse amplitude control of the first and second embodiments.

In particular, in some exemplary implementations, the CIED is an implantable cardiac pacemaker, or an implantable cardioverter-defibrillator (ICD). It should be noted that, for more details of the implantable cardiac pacemaker or ICD, reference is made to related techniques known to those skilled in the art to get a better understanding, and further description thereof is omitted for the sake of brevity and conciseness.

Of course, in other implementations, the CIED may also be an implantable cardiac pacemaker or other medical device designed for cardiac pacing or treatment by delivering pacing pulses thereto. Further description of these implementations is omitted for the sake of brevity and conciseness.

Reference throughout this specification to "one embodiment", "some embodiments", "an example", "a specific example" "some examples" or the like means that a particular feature, structure, material, or characteristic described in connection with the embodiments or examples is included in at least one embodiment or example of the invention. Thus, the appearances of those phrases in various places throughout this specification are not necessarily referring to the same embodiment or example of the invention. Furthermore, the particular features, structures, materials, or characteristics may be combined in any suitable manner in any one or more embodiments. Further, should there be no contradiction, one of ordinary skill in the art can combine the various embodiments, examples and features thereof described herein in any combination.

As can be seen from above, the methods for pacing pulse amplitude control and CIED of present invention offer the following benefits over the prior art.

The methods for pacing pulse amplitude control fully leverages the fact that the time constant of an RC charge circuit (e.g., consisting of a resistor in the charge optimization circuit and the first capacitor) is proportional to the product of the resistance and capacitance to charge the first capacitor through a smaller resistor when in an MR interfering environment, allowing the charging to be completed within a much shorter time and hence rendering the first capacitor more resistant to EMI in the MRI. As a result, during the pacing discharge of the cardiac implantable electronic device (specifically the first capacitor), the risk of a decrease in pacing pulse amplitude caused by an EMI in MRI is reduced and the health of a patient during an MRI check can better protected. In addition, the risk of incorrect diagnostics associated with attenuated pacing pulses from the CIED can be reduced, and successful cardiac pacing of the CIED can be ensured.

In the method for pacing pulse amplitude control, the discharge optimization circuit added to the pacing discharge circuit is of a novel design, which can provide appropriate quality factors Q for various scenarios by changing different resistors connected into a band-stop LC filter through controlling the states of associated switches. This addresses the contradiction between filtering effect of MR radio frequency and an impact on the amplitude of pacing pulses and allows pacing pulses to be delivered to the heart almost without being affected.

The CIED embodies the same inventive concept as, and therefore has at least all the advantages of, the methods for pacing pulse amplitude control. For more details of these advantages, reference is made to the above description in connection with the methods for pacing pulse amplitude control, and further description thereof is omitted for the sake of brevity and conciseness.

Various methods for pacing pulse amplitude control and CIED configurations disclosed herein have been described in detail above with respect to the foregoing embodiments. Of course, the above description is merely that of some preferred modes of carrying out the invention and is in no way intended to limit the scope thereof. Possible configurations of the present invention include, but are not limited to, those described in the foregoing embodiments, and those skilled in the art can obtain in light of the above teachings. Accordingly, any and all variations and modification made by those of ordinary skill in the art to which the present invention pertains in light of the above teachings are intended to fall within the scope thereof as defined by the appended claims.

## Claims

1. A method for pacing pulse amplitude control that is used in a cardiac implantable electronic device (CIED), wherein an equivalent circuit of the CIED comprises a contact impedance between an electrode lead and myocardial tissue, an electrode resistance of the electrode lead, a filter, a first capacitor, a discharge switch and a power supply, wherein the contact impedance, the electrode resistance, the filter, the first capacitor and the discharge switch are connected in series and form a pacing discharge circuit, wherein the first capacitor and the power supply are connected in series and form a pacing charge circuit, wherein the pacing charge circuit further comprises a charge optimization circuit connected in series with the power supply, wherein the charge optimization circuit comprises at least two parallel-connected charge optimization sub-circuits, wherein each charge optimization sub-circuit comprises a resistor and a switch that are connected in series, wherein resistances of the resistors in any two of the charge optimization sub-circuits are different, and
wherein the method comprises, in case of charging the first capacitor, opening the discharge switch and performing the steps of:
if the CIED is determined not to be in a magnetic resonance (MR) interfering environment, conducting at least one of the charge optimization sub-circuits having a higher resistance, and disconnecting the other charge optimization sub-circuit(s); and
if the CIED is determined to be in an MR interfering environment, conducting at least one of the charge optimization sub-circuits having a lower resistance, and disconnecting the other charge optimization sub-circuit(s).

2. The method according to claim 1, wherein the charge optimization circuit comprises two parallel-connected charge optimization sub-circuits, wherein a first charge optimization sub-circuit comprises a first resistor and a first switch that are connected in series, wherein a second charge optimization sub-circuit comprises a second resistor and a second switch that are connected in series, wherein the first resistor has a higher resistance than the second resistor, wherein the method comprises, in case of charging the first capacitor, opening the discharge switch and performing the steps of:
if the CIED is determined not to be in an MR interfering environment, closing the first switch and opening the second switch, thereby forming the pacing charge circuit that is in a conductive state by the power supply, the first switch, the first resistor and the first capacitor, wherein the power supply charges the first capacitor through the first resistor; and
if the CIED is determined to be in an MR interfering environment, closing the second switch and opening the first switch, thereby forming the pacing charge circuit that is in a conductive state by the power supply, the second switch, the second resistor and the first capacitor, wherein the power supply charges the first capacitor through the second resistor.

3. The method according to claim 2, wherein a resistance of the first resistor is in a range of 4 MΩ to 6 MΩ, and wherein a resistance of the second resistor is in a range of 100 Ω to 200 Ω.

4. The method according to claim 1, wherein a first terminal of the charge optimization circuit is connected to a first terminal of the filter and a first terminal of first capacitor, and wherein a second terminal of the charge optimization circuit is electrically connected to a positive terminal of the power supply.

5. The method according to claim 1, further comprising, subsequent to the charging of the first capacitor, conducting the pacing discharge circuit and disconnecting the pacing charge circuit, thereby allowing the first capacitor to deliver a pacing pulse.

6. The method according to claim 5, wherein the filter comprises a second capacitor, a discharge optimization circuit and an inductor, wherein the second capacitor and the discharge optimization circuit are connected in series and then are connected in parallel to the inductor, wherein the discharge optimization circuit comprises at least two parallel-connected discharge optimization sub-circuits, wherein each discharge optimization sub-circuit comprises a resistor and a switch that are connected in series, wherein resistances of the resistors in any two of the discharge optimization sub-circuits are different, and
wherein the method comprises, when the first capacitor is in a non-charging state, disconnecting the charge optimization circuit and performing the steps of:
if the CIED is determined not to be in an MR interfering environment, disconnecting the discharge optimization circuit and closing a discharge switch, wherein the first capacitor delivers a pacing pulse through the inductor and the second capacitor;
if the CIED is determined to be in an MR interfering environment and a pacing pulse delivery is not needed, conducting at least one of the discharge optimization sub-circuits having a higher resistance and opening the discharge switch and the other discharge optimization sub-circuit(s), wherein the second capacitor confines radio frequency of an interfering signal source within the filter through the conducted discharge optimization sub-circuit and the inductor; and
if the CIED is determined to be in an MR interfering environment and a pacing pulse delivery is needed, conducting at least one of the discharge optimization sub-circuits having a lower resistance and the discharge switch, and disconnecting the other discharge optimization sub-circuit(s), wherein the first capacitor delivers a pacing pulse through the conducted discharge optimization sub-circuit and the second capacitor.

7. The method according to claim 6, wherein the discharge optimization circuit comprises two parallel-connected discharge optimization sub-circuits, wherein a first discharge optimization sub-circuit comprises a third resistor and a third switch that are connected in series, wherein a second discharge optimization sub-circuits comprises a fourth resistor and a fourth switch that are connected in series, wherein the third resistor has a higher resistance than the fourth resistor, and
wherein the method comprises, when the first capacitor is in a non-charging state, disconnecting the charge optimization circuit and performing the steps of:
if the CIED is determined not to be in an MR interfering environment, opening the third and fourth switches and closing the discharge switch, thereby forming the pacing discharge circuit that is in a conductive state by the first capacitor, the inductor, the second capacitor, the electrode resistance, the contact impedance and the discharge switch, wherein the first capacitor delivers a pacing pulse through the inductor and the second capacitor;
if the CIED is determined to be in an MR interfering environment and a pacing pulse delivery is not needed, closing the third switch and opening the fourth and discharge switches, thereby forming the pacing discharge circuit that is in a conductive state by the second capacitor, the inductor, the third switch and the third resistor, wherein the second capacitor confines radio frequency of the interfering signal source within the filter through the third resistor and the inductor; and
if the CIED is determined to be in an MR interfering environment and a pacing pulse delivery is needed, closing the fourth and discharge switches and opening the third switch, thereby forming the pacing discharge circuit that is in a conductive state by the first capacitor, the inductor, the fourth switch, the fourth resistor, the second capacitor, the electrode resistance, the contact impedance and the discharge switch, wherein the first capacitor delivers a pacing pulse through the fourth resistor and the second capacitor.

8. The method according to claim 7, wherein a resistance of the third resistor is in a range of 1 MΩ to 5 MΩ, and wherein a resistance of the fourth resistor is in a range of 100 Ω to 500 Ω.

9. The method according to claim 6, wherein a first terminal of the discharge optimization circuit is connected to each of a first terminal of the inductor, a first terminal of the charge optimization circuit and a first terminal of first capacitor, wherein a second terminal of the discharge optimization circuit is connected to a first terminal of the second capacitor, and wherein a second terminal of the inductor is connected between the electrode resistance and the second capacitor.

10. The method according to claim 1, wherein the filter is any one of a band-stop filter, a passive filter, an active filter and a digital filter.

11. The method according to claim 1, wherein a resonant frequency of the filter is equal to an MRI radio frequency.

12. A cardiac implantable electronic device (CIED), using the method for pacing pulse amplitude control according to any one of claims 1 to 11 for controlling the pacing pulse.

13. The CIED according to claim 12, implemented as an implantable cardiac pacemaker, or as an implantable cardioverter-defibrillator (ICD).

14. A cardiac implantable electronic device (CIED), comprising a contact impedance between an electrode lead and myocardial tissue, an electrode resistance of the electrode lead, a filter, a first capacitor, a discharge switch and a power supply, wherein the contact impedance, the electrode resistance, the filter, the first capacitor and the discharge switch are connected in series and form a pacing discharge circuit, wherein the first capacitor and the power supply are connected in series and form a pacing charge circuit, wherein the pacing charge circuit further comprises a control unit and a charge optimization circuit connected in series with the power supply, wherein the charge optimization circuit comprises at least two parallel-connected charge optimization sub-circuits, wherein each charge optimization sub-circuit comprises a resistor and a switch that are connected in series, wherein resistances of the resistors in any two of the charge optimization sub-circuits are different, wherein the control unit is configured to control pacing pulse amplitude depending on whether the CIED is in an MR interfering environment, thereby avoiding or reducing MR interference.

15. The CIED according to claim 14, wherein the control unit is configured to implement the method for pacing pulse amplitude control according to any one of claims 1 to 11.
